# EUROPEAN PATENT SPECIFICATION

(11) **EP 1 656 180 B1**
(45) Date of publication and mention of the grant of the patent: **18.04.2012**
(21) Application number: 04779566.1
(22) Date of filing: 28.07.2004
(51) Int. Cl.: A61N 1/05

(54) **APPARATUS FOR IMPLANTING AN ELECTRICAL STIMULATION SYSTEM AND A PADDLE STYLE ELECTRICAL STIMULATION LEAD**
GERÄT ZUR IMPLANTATION EINES ELEKTRISCHEN STIMULATIONSSYSTEMS UND ELEKTRISCHE STIMULATIONSLEITUNG VOM PADDEL-TYP
APPAREIL PERMETTANT D'IMPLANTER UN SYSTEME DE STIMULATION ELECTRIQUE ET UNE DERIVATION DE STIMULATION ELECTRIQUE DE STYLE PALETTE

(30) Priority: 08.08.2003 US 637342
(43) Date of publication of application: 17.05.2006
(60) Divisional application: 12157400.8
(73) Proprietor: ADVANCED NEUROMODULATION SYSTEMS, INC., Plano, TX 75024 (US)
(72) Inventor: DAGLOW, Terry, Allen, TX 75013 (US)
(74) Representative: Lawrence, John
(86) International application number: PCT/US2004/024552
(87) International publication number: WO 2005/016447

(56) References cited:
- WO-A-03/013650
- US-A1- 2002 147 485

## Description

### TECHNICAL FIELD OF THE INVENTION

This invention relates generally to electrical stimulation leads for medical applications and in particular to an apparatus for implanting an electrical stimulation system that includes a paddle style electrical stimulation lead.

### BACKGROUND

Electrical energy is applied to the spinal cord and peripheral nerves to treat regions of the body that are affected by chronic pain from a variety of etiologies. One method of delivering electrical energy is to implant an electrode and position it in a precise location adjacent the spinal cord such that stimulation of the electrode causes a subjective sensation of numbness or tingling in the affected region of the body, known as "paresthesia." Pain managing electrical energy is commonly delivered through electrodes positioned external to the dura layer surrounding the spinal cord. The electrodes may be carried by either of two primary vehicles: a percutaneous lead and a laminotomy or "paddle" lead.

Percutaneous leads commonly have three or more equally-spaced electrodes. They are positioned above the dura layer using a needle that is passed through the skin, between the desired vertebrae and onto the top of the dura. Percutaneous leads deliver energy radially in all directions because of the circumferential nature of the electrode. Percutaneous leads can be implanted using a minimally invasive technique. In a typical percutaneous lead placement, a trial stimulation procedure is performed to determine the optimal location for the lead. Here, a needle is placed through the skin and between the desired vertebrae. The percutaneous lead is then threaded through the needle into the desired location over the spinal cord dura. Percutaneous leads may also be positioned in other regions of the body near peripheral nerves for the same purpose.

Laminotomy or paddle style leads have a paddle-like configuration and typically possess multiple electrodes arranged in one or more independent columns. Paddle style leads provide a more focused energy delivery than percutaneous leads because electrodes may be present on only one surface of the lead. Paddle style leads may be desirable in certain situations because they provide more direct stimulation to a specific surface and require less energy to produce a desired effect. Because paddle style leads are larger than percutaneous leads, they have historically required surgical implantation through a procedure known as partial laminectomy that requires the resection and removal of vertebral tissue.

The invention is defined in claims 1 and 19 Document US-A-2002/147485 discloses the most relevant prior art.

### BRIEF DESCRIPTION OF THE DRAWINGS

To provide a more complete understanding of the present invention and the features and advantages thereof, reference is made to the following description taken in conjunction with the accompanying drawings, in which:
FIGURE 1A illustrates an example introducer for implanting a paddle style electrical stimulation lead;
FIGURE 1B illustrates an example inner penetrator of an introducer for implanting a paddle style electrical stimulation lead;
FIGURE 1C illustrates an example of an outer sheath of an introducer for implanting a paddle style electrical stimulation lead;
FIGURE 1D illustrates an example of a tip of an introducer for implanting a paddle style electrical stimulation lead;
FIGURE 1E illustrates an example of a tip of an outer sheath of an introducer for implanting a paddle style electrical stimulation lead;
FIGURE 1F illustrates a side view of an example of a tip of an introducer for implanting a paddle style electrical stimulation lead;
FIGURE 2A illustrates an example of a needle inserted into a human's epidural space;
FIGURE 2B illustrates an example of a guide wire being inserted through a needle into a human's epidural space;
FIGURE 2C illustrates an example of an introducer being inserted over a guide wire into a human's epidural space;
FIGURE 2D illustrates an example of an inner penetrator being removed from the outer sheath of an introducer in a human's epidural space;
FIGURE 2E illustrates an example of a paddle style lead being inserted through an introducer into a human's epidural space;
FIGURE 2F illustrates an example of a paddle style lead implanted in a human's epidural space;
FIGURE 3A illustrates an example of a stimulation system;
FIGURE 3B illustrates an example of a stimulation system; and
FIGURE 4 is a flow chart describing steps for implanting a stimulation system.

### DESCRIPTION OF EXAMPLE EMBODIMENTS

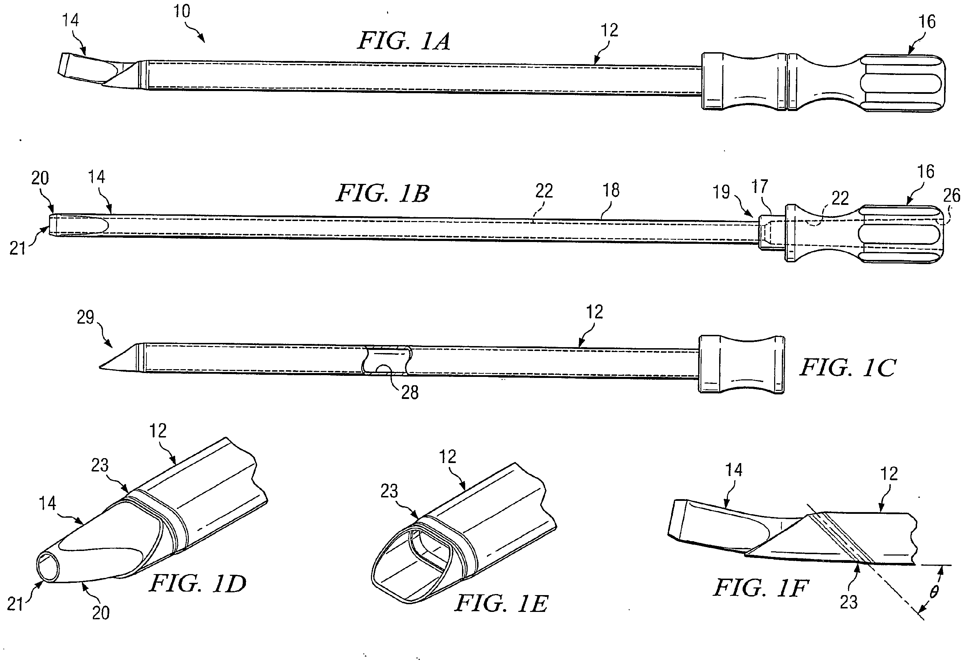

FIGURE 1A illustrates an example introducer 10 for implanting a paddle style electrical stimulation lead percutaneously. Introducer 10 may be used to percutaneously introduce a percutaneous or paddle style lead into the epidural space of a user who requires electrical stimulation treatment directed to spinal nerve tissue, for example, for pain management. As known in the art, paddle style leads generally have a width about two times the height of the face of the paddle. The same or an analogous, perhaps smaller, introducer 10 may be used to implant a percutaneous or paddle style lead into other tissue for electrostimulation treatment of a peripheral nerve. In one embodiment, introducer 10 includes an outer sheath 12 and an inner penetrator 14.

FIGURE 1B illustrates an example inner penetrator 14 disassembled from outer sheath 12. Inner penetrator 14 includes handle 16, connector 17, and body 18 having proximal end 19 and distal end or tip 20. Tip 20 may be tapered. Connector 17 connects handle 16 to body 18. An inner channel 22 is formed through handle 16 and body 18 and connects opening 26 of handle 16 to opening 21 of tip 20. Inner channel 22 may be configured to attach to a syringe. Inner channel 22 is wide enough to accommodate guide wires of various sizes along which introducer 10 may be advanced during use. Channel 22 may taper or otherwise decrease in diameter as it traverses connector 17 at the handle-body junction. Inner penetrator 14 may be formed from a plastic, such as silastic or another polymer, or any other suitable material. Tip 20 of inner penetrator 14 is curved into a suitable configuration to allow passage around an anatomical obstruction .

FIGURE 1C illustrates outer sheath 12 disassembled from inner penetrator 14. The lumen of outer sheath 12 may range in width, for example from approximately 2mm to approximately 6mm. The lumen may be oblong, oval, or substantially rectangular as needed to accommodate paddle style leads of various configurations. Outer sheath 12 may taper slightly at tip 29. Tip 29 of outer sheath 12 may be beveled to allow easier passage through tissue and to allow inner penetrator 14 to protrude out of tip 29.

Outer sheath 12 is preferably formed from a metal, such as stainless steel or titanium, or any other suitable material that is stiff and resists bending when outer sheath 12 is inserted through the paravertebral tissue and into the epidural space. In one embodiment, inner penetrator 14 includes tapered tip 20 shown in FIGURE 1D. Tapered tip 20 protrudes out of outer sheath 12. Tapered tip 20 preferably allows introducer 10 to pass easily over a guide wire without creating a false passage in an undesirable location in the tissue.

In one embodiment of outer sheath 12, shown in FIGURES 1D-F, tip 20 includes a raised circumferential shoulder or ridge 23 configured to provide an indication or "feel" to a physician as raised ridge 23 comes in contact with the ligamentum flavum. This "feel" occurs when raised ridge 23 comes in contact with the ligamentum flavum causing a slight resistance, pressure, or "notch" feel to the physician as raised ridge 23 comes in contact with and passes through the ligamentum flavum. As many physicians rely on "feel" while performing delicate procedures, this aspect may provide an important indication to the physician as to the location of outer sheath 12 and thus introducer 10 as a whole.

Such a raised ridge 23 can also be applied to needles or cutting devices that otherwise fail to provide physicians sufficient "feel" or a locative indication as the needle cuts through the ligamentum flavum. For example, the edge of outer sheath 12 in FIGURE 1E could be configured into a cutting surface for a paddle insertion type needle. The improvement of raised ridge 23 on such a cutting device would provide the needed "feel" or indication to the physician as to where the needle was in the human tissue, thus providing confidence to the physician, as the physician uses such a large needle, that the needle has not yet entered the interthecal space.

Further, raised ridge 23 assists in spreading the fibers of the paravertebral muscle and ligaments as it is inserted. Raised ridge 23 may be angled to assist insertion, for example, at an angle of thirty-five to forty-five degrees or any other angle that would facilitate passage of outer sheath through tissue. During insertion, raised ridge 23 ultimately makes contact with the ligamentum flavum and rests against it during insertion of a guide wire and an electrical stimulation lead.

In one embodiment, outer sheath 12, inner penetrator 14, or both may be formed from radio-opaque material or may include radio-opaque markers that allow the position of outer sheath 12, inner penetrator 14, or both to be visualized with fluoroscopy or plain x-rays, for example, during the insertion process to insure proper positioning in the epidural space.

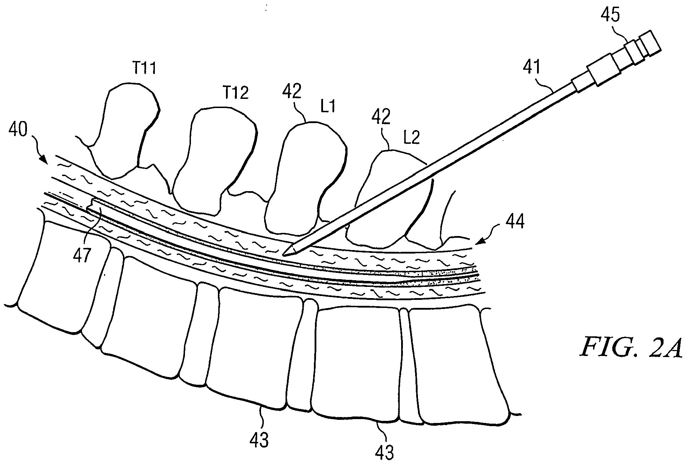

FIGURES 2A-F illustrate an example method of implanting a paddle style electrical stimulation lead into a human's epidural space using an example introducer 10. Spinal cord 47 is also shown. A location between two vertebrae is selected for the procedure. The site may be selected using fluoroscopy. The first step in performing the procedure is to insert needle 41, preferably at an angle, into the skin, and through the subcutaneous tissue and ligamentum flavum 44 of the spine, and into a human's epidural space 40. In one embodiment of the method, for example, the introducer might be inserted at an angle of approximately thirty-five to approximately forty-five degrees. FIGURE 2A illustrates insertion of needle 41 through the skin between spinous processes 42 of two vertebrae 43. Entry into epidural space 40 by needle 41 may be confirmed using standard methods such as the "loss-of-resistance" technique after stylet 45, or inner portion of needle 41, is removed.

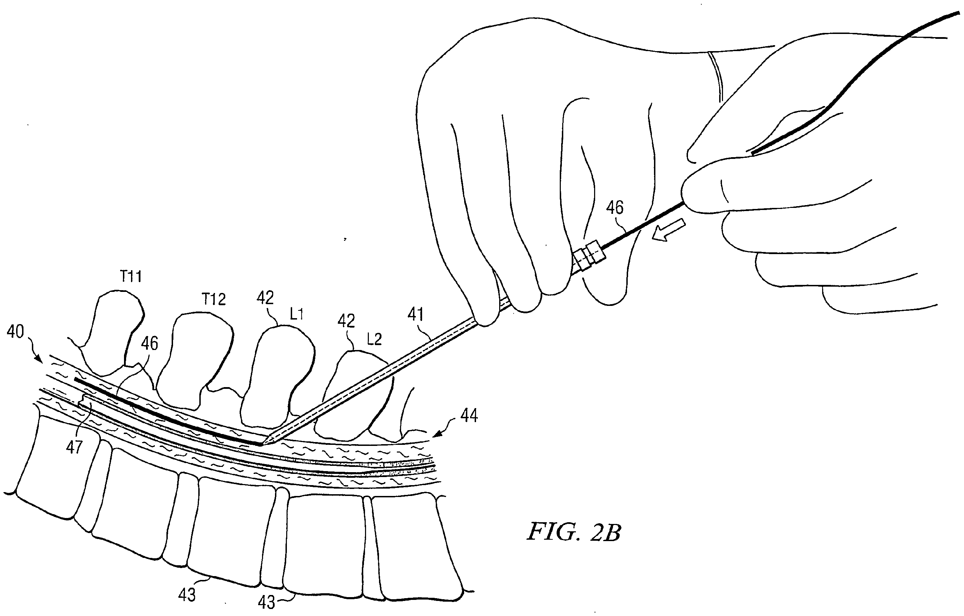

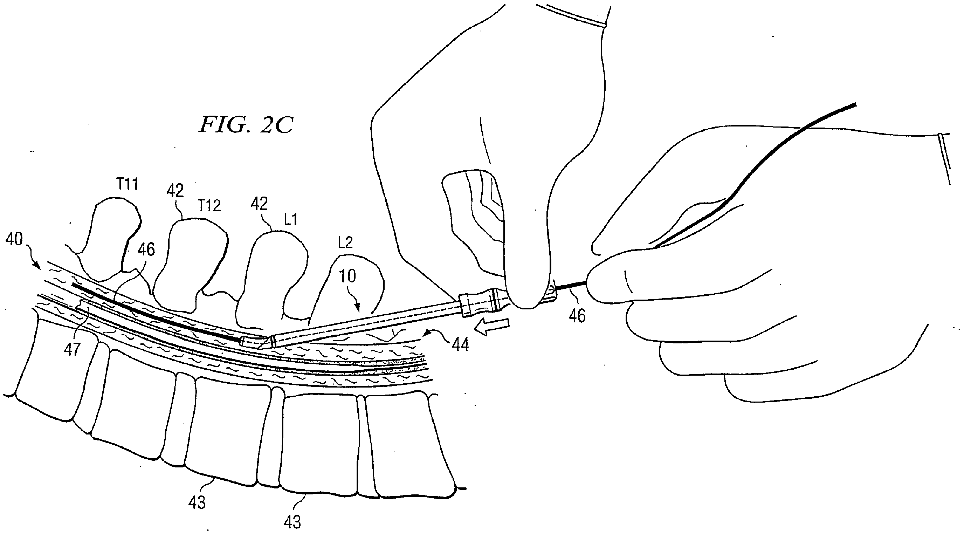

After removing stylet 45 from needle 41, guide wire 46 may be inserted through needle 41 into epidural space 40, shown in FIGURE 2B. A guide wire is used in a preferred embodiment of the method of insertion but is not required to insert a paddle style lead through the introducer. This part of the procedure may be performed under fluoroscopic guidance for example. Fluoroscopy may be used to check the position of guide wire 46 in epidural space 40 before inserting introducer 10. Once the tip of guide wire 46 is within epidural space 40, needle 41 is removed. As shown in FIGURE 2C, introducer 10 may then be inserted, preferably at an angle of approximately thirty-five to approximately forty-five degrees, although the exact angle may differ depending on technique and a patient's anatomy, over guide wire 46 and into epidural space 40 using guide wire 46 as a guide. The technique of passing introducer 10 over guide wire 46 helps ensure proper placement of introducer 10 into epidural space 40 and helps avoid inadvertent passage of introducer 10 into an unsuitable location. The operator may choose to cut the skin around the insertion site with a scalpel to facilitate subsequent entry of introducer 10 through the needle entry site.

As introducer 10 is passed through the skin it elongates the hole in the skin made by needle 41. As introducer 10 is passed deeper into the paravertebral tissues, it spreads the fibers of tissue, muscle and ligamentum flavum 44 and forms a tract through these tissues and into epidural space 40, preferably without cutting the tissues. At the level in the tissues where introducer 10 meets and penetrates ligamentum flavum 44 there is a second loss of resistance when inner penetrator 14 has completely penetrated the ligamentum flavum 44. Shoulder or ridge 23 of outer sheath 12 is preferably lodged against ligamentum flavum 44 during insertion of a paddle style lead.

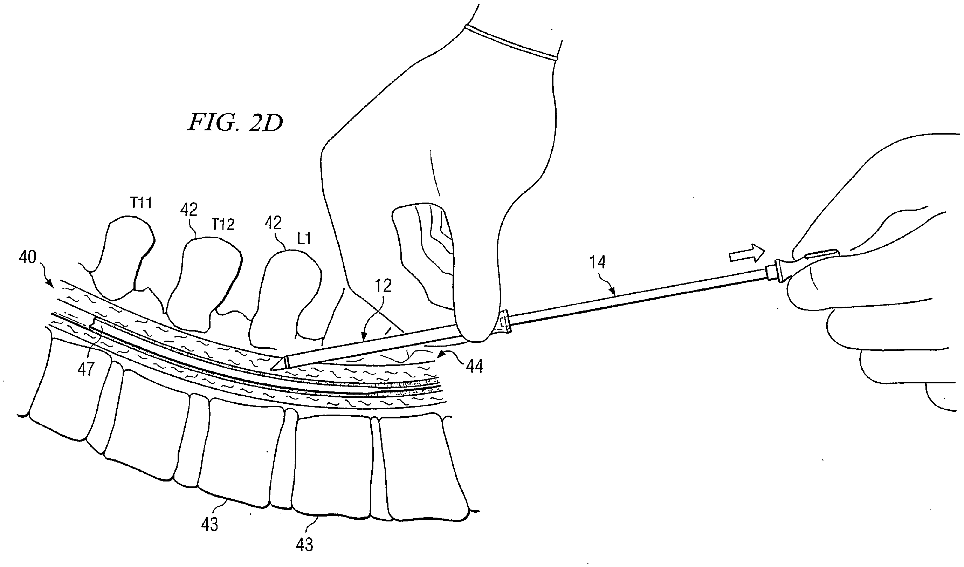

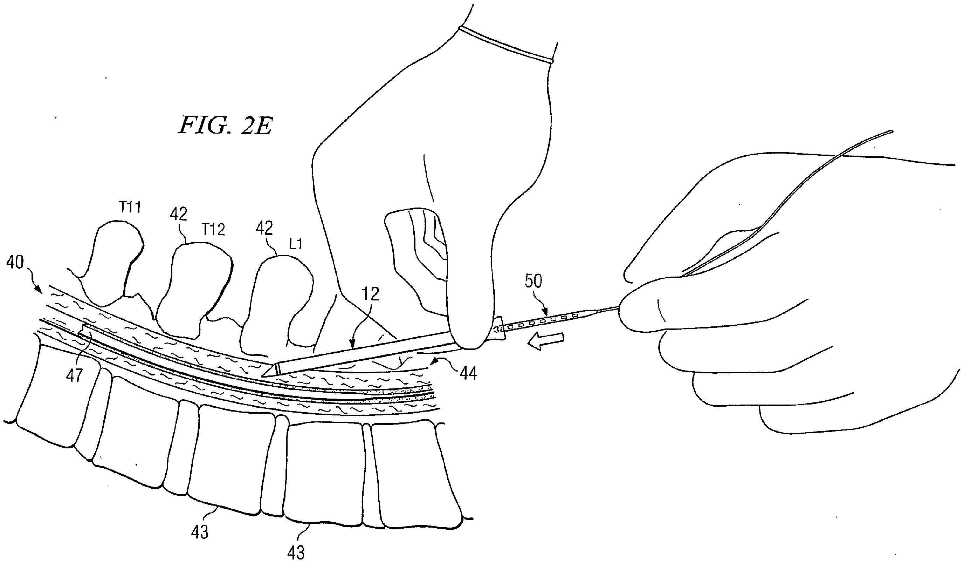

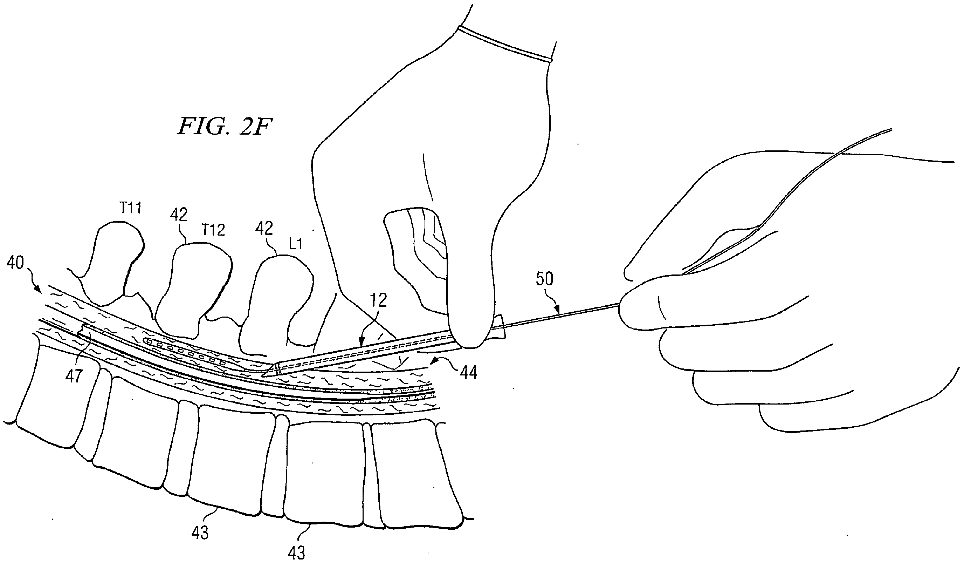

Once introducer 10 has completely penetrated ligamentum flavum, inner penetrator 14 and guide wire 46 may be removed, leaving outer sheath 12 positioned in epidural space 40, as shown in FIGURE 2D. As shown in FIGURE 2E, paddle style lead 50 may then be inserted through outer sheath 12 and positioned at an optimal vertebral level, using fluoroscopy for example, for the desired therapeutic effect. As shown in FIGURE 2F, outer sheath 12 may then be removed leaving only paddle style lead 50 in epidural space 40, where paddle style lead 50 can be further manipulated if necessary to achieve a desired therapeutic effect. Paddle style lead 50 may be secured by suturing it to a spinous process.

As described above, introducer 10 may be used to implant paddle style lead 50 into epidural space 40 for spinal nerve stimulation. The same or an analogous, perhaps smaller, introducer 10 may be used to implant an analogous paddle style lead 50 into any appropriate region of the body for peripheral nerve stimulation. For example, such a paddle style lead 50 may have an outer sheath 12 and lumen 28 with a width of approximately 1mm to approximately 3mm.

A similar method of insertion (not expressly shown) may be used to implant a paddle style electrical stimulation lead into a human's peripheral nerve tissue. In this embodiment a site for insertion in tissue near a nerve is selected. The first step in performing the procedure is to insert a needle into the skin and through the subcutaneous tissue and into tissue near a peripheral nerve. If the needle has a stylet, it may be removed and a guide wire may be inserted through the needle and into the tissue near a peripheral nerve. A guide wire may not be required. Fluoroscopy may or may not be used to guide insertion of a guide wire into tissue near a peripheral nerve. Once the tip of the guide wire, or needle, is in the tissue near a peripheral nerve, introducer 10 may be inserted, preferably at an angle that would depend on the anatomy of the body near the peripheral nerve to be stimulated. As introducer 10 is passed through tissues, it elongates the tract made by a needle or guide wire and spreads the tissue. After positioning introducer 10 in tissue adjacent to the peripheral nerve to be stimulated, inner penetrator 14 is removed. A paddle style lead may then be inserted through outer sheath 12. Outer sheath 12 may then be removed leaving only the paddle style lead in position near the peripheral nerve to be stimulated.

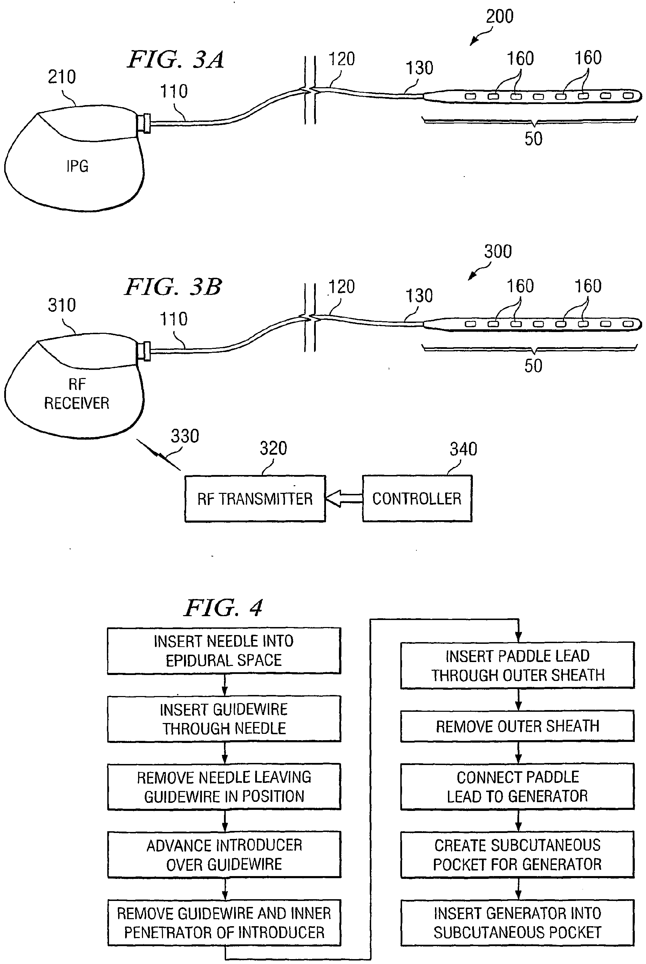

Now referring to FIGURES 3A and 3B, there are shown two embodiments of a stimulation system 200, 300. The stimulation systems generate and apply a stimulus to a tissue or to a certain location of a body. In general terms, the system 200, 300 includes a stimulation or energy source 210, 310 and a lead 50 for application of the stimulus. The lead 110 shown in FIGURES 3A and 3B is the paddle style lead 50.

As shown in FIGURE 3A, the stimulation system 200 includes the lead 50 that is coupled to the stimulation source 210. In one embodiment, the stimulation source 210 includes an implantable pulse generator (IPG). As is known in the art, an implantable pulse generator (IPG) is implanted within the body (not shown) that is to receive electrical stimulation from the stimulation source 210. An example IPG may be one manufactured by Advanced Neuromodulation Systems, Inc., such as the Genesis® System, part numbers 3604, 3608, 3609, and 3644.

As shown in FIGURE 3B, the stimulation system 300 includes the lead 50 that is coupled to the stimulation source 310. The stimulation source 310 includes a wireless receiver. As is known in the art, the stimulation source 310 comprising a wireless receiver is implanted within the body (not shown) that is to receive electrical stimulation from the stimulation source 310. An example wireless receiver 310 may be those wireless receivers manufactured by Advanced Neuromodulation Systems, Inc., such as the Renew® System, part numbers 3408 and 3416.

The wireless receiver (not shown) within stimulation source 310 is capable of receiving wireless signals from a wireless transmitter 320. The wireless signals are represented in FIGURE 3B by wireless link symbol 330. The wireless transmitter 320 and a controller 340 are located outside of the body that is to receive electrical stimulation from the stimulation source 310. A user of the stimulation source 310 may use the controller 340 to provide control signals for the operation of the stimulation source 310. The controller 340 provides control signals to the wireless transmitter 320. The wireless transmitter 320 transmits the control signals (and power) to the receiver in the stimulation source 310 and the stimulation source 310 uses the control signals to vary the signal parameters of the electrical signals that are transmitted through lead 110 to the stimulation site. An example wireless transmitter 320 may be those transmitters manufactured by Advanced Neuromodulation Systems, Inc., such as the Renew® System, part numbers 3508 and 3516.

As will be appreciated, the connectors are not visible in FIGURES 3A and 3B because the contact electrodes are situated within a receptacle (not shown) of the stimulation source 210, 310. The connectors are in electrical contact with a generator (not shown) of electrical signals within the stimulation source 210, 310. The stimulation source 210, 310 generates and sends electrical signals via the lead 50 to the electrodes 160. Understandably, the electrodes 160 are located at a stimulation site (not shown) within the body that is to receive electrical stimulation from the electrical signals. A stimulation site may be, for example, adjacent to one or more nerves in the central nervous system (e.g., spinal cord) or peripheral nerves. The stimulation source 210, 310 is capable of controlling the electrical signals by varying signal parameters (e.g., intensity, duration, frequency) in response to control signals that are provided to the stimulation source 210, 310.

As described above, once lead 110 is inserted into either the epidural space or near the peripheral nerve, introducer 10 is removed. Lead 110 extends from the insertion site to the implant site (the area of placement of the generator). The implant site is typically a subcutaneous pocket that receives and houses the IPG or receiver (providing stimulation source 210, 310). The implant site is usually positioned a distance away from the stimulation site, such as near the buttocks or other place in the torso area. In most cases, the implant site (and insertion site) is located in the lower back area, and lead 110 may extend through the epidural space (or other space) in the spine to the stimulation site (e.g., middle or upper back, neck, or brain areas). Once the system is implanted, the system of leads and/or extensions may be subject to mechanical forces and movement in response to body movement. FIGURE 4 illustrates the steps that may be used to implant a stimulation system 200, 300 into a human.

Although the present invention has been described with several embodiments, a number of changes, substitutions, variations, alterations, and modifications may be suggested to one skilled in the art, and it is intended that the invention encompass all such changes, substitutions, variations, alterations, and modifications as fall within the scope of the appended claims.

## Claims

1. An introducer for implanting an electrical stimulation lead to enable electrical stimulation of nerve tissue, comprising:
an outer sheath configured to accommodate insertion of the electrical stimulation lead through the outer sheath, the outer sheath operable to be inserted into a human body near the nerve tissue; and
an inner penetrator removably housed within the outer sheath and comprising an inner channel configured to accommodate a guide wire, the inner penetrator configured to be advanced along the guide wire to a desired location relative to the nerve tissue and removed from the outer sheath leaving the outer sheath substantially in position for insertion of the electrical stimulation lead through the outer sheath into position proximate the nerve tissue, **characterized in that** the tip of the inner penetrator and the tip of the outer sheath comprise a curved portion to allow passage of the inner penetrator and outer sheath around an anatomical obstruction.

2. The introducer of Claim 1, wherein the lead is greater than 1.5 millimeters in width.

3. The introducer of Claim 1, wherein the electrical stimulation lead has a width of at least approximately two times its height.

4. The introducer of Claim 1, wherein the electrical stimulation lead is a paddle style lead.

5. The introducer of Claim 1, wherein the nerve tissue comprises spinal nerve tissue and the desired location comprises an epidural space of the human.

6. The introducer of Claim 1, wherein the nerve tissue comprises a peripheral nerve.

7. The introducer of Claim 1, wherein the inner penetrator comprises a hollow tip configured to extend beyond the outer sheath.

8. The introducer of Claim 1, wherein the outer sheath has a raised circumferential ridge configured to create resistance when the circumferential ridge contacts the human's ligamentum flavum.

9. The introducer of Claim 1, wherein the inner penetrator and outer sheath comprise one or more radio-opaque markers for visualization using fluoroscopy.

10. The introducer of Claim 1, wherein the outer sheath has a substantially oval cross-section.

11. The introducer of Claim 1, wherein the outer sheath has a substantially oblong cross-section.

12. The introducer of Claim 1, wherein the outer sheath is formed of metal comprising titanium or stainless steel.

13. The introducer of Claim 1, wherein the inner penetrator is formed from one or more of plastic, silastic, or a polymeric material.

14. The introducer of Claim 1, wherein the tip of the outer penetrator is tapered.

15. The introducer of Claim 1, wherein the inner penetrator is configured to be advanced until an end of the inner penetrator is positioned in the human's epidural space at a desired location relative to the nerve tissue such that the outer sheath forms an insertion tract for the electrical stimulation lead as the inner penetrator advances along the guide wire.

16. The introducer of Claim 1, wherein the tip of the introducer is tapered and hollow.

17. The introducer of Claim 1, wherein the outer sheath comprises a circumferential ridge configured to create resistance when the circumferential ridge contacts a ligamentum flavum of a human.

18. The introducer of Claim 1, wherein the needle comprises a removable stylet configured to be removed before inserting the guide wire.

19. A system for implanting an electrical stimulation lead to enable electrical stimulation of a human's spinal nerve tissue, comprising:
a needle;
a guide wire;
an introducer comprising an outer sheath and an inner penetrator;
the outer sheath configured to accommodate insertion of the electrical stimulation lead through the outer sheath, the outer sheath operable to be inserted through the human's skin and into the human's epidural space;
an inner penetrator removably housed within the outer sheath and comprising an inner channel configured to accommodate a guide wire;
the inner penetrator configured to be advanced along the guide wire until an end of the inner penetrator is positioned in the epidural space at a desired location relative to spinal nerve tissue to be stimulated such that the outer sheath forms an insertion tract for the electrical stimulation lead as the inner penetrator advances along the guide wire, the inner penetrator configured to be removed from the outer sheath leaving the outer sheath substantially in position in the epidural space for insertion of the electrical stimulation lead through the outer sheath into position proximate the spinal nerve tissue to be stimulated; and an implantable generator to power the electrical stimulation lead,
**characterized in that** the tip of the inner penetrator and the tip of the outer sheath comprise a curved portion to allow passage of the inner penetrator and outer sheath around an anatomical obstruction.

## Patentansprüche

1. Eine Einführhilfe zur Implantation einer elektrischen Stimulationsleitung, um die elektrische Stimulation des Nervengewebes zu ermöglichen, bestehend aus:
einem Außenmantel, der so beschaffen ist, dass die Einführung der elektrischen Stimulationsleitung durch den Außenmantel ermöglicht wird, wobei der Außenmantel einsetzbar ist, um in den menschlichen Körper in der Nähe des Nervengewebes eingeführt zu werden; und
einem inneren Eindringkörper, der herausnehmbar innerhalb des Außenmantels untergebracht ist und einen Innenkanal umfasst, der dazu dient, einen Führungsdraht aufzunehmen, wobei der innere Eindringkörper so gestaltet ist, dass er entlang dem Führungsdraht zu einem gewünschten Ort relativ zum Nervengewebe vorgeschoben und vom Außenmantel entfernt werden kann, wodurch der Außenmantel im Wesentlichen in der Stellung belassen wird, um die elektrische Stimulationsleitung durch den Außenmantel in eine Position in der Nähe des Nervengewebes einzuführen, **dadurch gekennzeichnet, dass** die Spitze des inneren Eindringkörpers und die Spitze des Außenmantels einen gekrümmten Teil beinhalten, um den Durchgang des inneren Eindringkörpers und des Außenmantels um ein anatomisches Hindernis herum zu ermöglichen.

2. Die Einführhilfe nach Anspruch 1, worin die Leitung mehr als 1,5 ml breit ist.

3. Die Einführhilfe nach Anspruch 1, worin die elektrische Stimulationsleitung ungefähr mindestens zweimal so breit wie lang ist.

4. Die Einführhilfe nach Anspruch 1, worin die elektrische Stimulationsleitung vom Paddel-Typ ist.

5. Die Einführhilfe nach Anspruch 1, worin das Nervengewebe Rückenmarknervengewebe umfasst und die gewünschte Stelle einen Epiduralraum beim Menschen beinhaltet.

6. Die Einführhilfe nach Anspruch 1, worin das Nervengewebe einen peripheren Nerv umfasst.

7. Die Einführhilfe nach Anspruch 1, worin der innere Eindringkörper eine hohle Spitze umfasst, die so gestaltet ist, dass sie sich über den Außenmantel hinaus ausdehnt.

8. Die Einführhilfe nach Anspruch 1, worin der Außenmantel einen umlaufenden erhöhten Rand aufweist, der dazu dient, einen Widerstand zu bieten, wenn der umlaufende erhöhte Rand beim Menschen mit dem Ligamentum flavum in Berührung kommt.

9. Die Einführhilfe nach Anspruch 1, worin der innere Eindringkörper und der Außenmantel einen oder mehrere strahlenundurchlässige Marker zur Visualisierung mit Hilfe der Fluoroskopie umfassen.

10. Die Einführhilfe nach Anspruch 1, worin der Außenmantel einen überwiegend ovalen Querschnitt aufweist.

11. Die Einführhilfe nach Anspruch 1, worin der Außenmantel einen überwiegend rechteckigen Querschnitt aufweist.

12. Die Einführhilfe nach Anspruch 1, worin der Außenmantel aus Metall gebildet ist, das Titan und Edelstahl beinhaltet.

13. Die Einführhilfe nach Anspruch 1, worin der innere Eindringkörper aus einem oder mehreren von einem Plastik-, Silastic- oder Polymermaterial gebildet ist.

14. Die Einführhilfe nach Anspruch 1, worin die Spitze des äußeren Eindringkörpers abgeschrägt ist.

15. Die Einführhilfe nach Anspruch 1, worin der innere Eindringkörper so gestaltet ist, dass er vorgeschoben werden kann, bis ein Ende des inneren Eindringkörpers an einer gewünschten Stelle des menschlichen Epiduralraumes relativ zum Nervengewebe positioniert ist, sodass der Außenmantel einen Gang zum Einführen der elektrischen Stimulationsleitung bildet, wenn sich der innere Eindringkörper entlang dem Führungsdraht nach vorne bewegt.

16. Die Einführhilfe nach Anspruch 1, worin die Spitze der Einführhilfe abgeschrägt und hohl ist.

17. Die Einführhilfe nach Anspruch 1, worin der Außenmantel einen umlaufenden erhöhten Rand umfasst, der dazu dient, einen Widerstand zu bieten, wenn der umlaufende erhöhte Rand beim Menschen mit dem Ligamentum flavum in Berührung kommt.

18. Die Einführhilfe nach Anspruch 1, worin die Nadel einen abnehmbaren Mandrin umfasst, der so gestaltet ist, dass er vor dem Einführen des Führungsdrahtes entfernt wird.

19. Ein System zur Implantation einer elektrischen Stimulationsleitung, um die elektrische Stimulation des menschlichen Rückenmarknervengewebes zu ermöglichen, bestehend aus:
einer Nadel;
einem Führungsdraht;
einer Einführhilfe mit einem Außenmantel und einem inneren Eindringkörper;
wobei der Außenmantel so beschaffen ist, dass die Einführung der elektrischen Stimulationsleitung durch den Außenmantel ermöglicht wird und der Außenmantel funktionsfähig ist, um durch die menschliche Haut und in den menschlichen Epiduralraum eingeführt zu werden;
einem inneren Eindringkörper, der herausnehmbar innerhalb des Außenmantels untergebracht ist und einen Innenkanal umfasst, der imstande ist, einen Führungsdraht aufzunehmen;
wobei der innere Eindringkörper so beschaffen ist, dass er entlang dem Führungsdraht vorgeschoben werden kann, bis ein Ende des inneren Eindringkörpers an einer gewünschten Stelle des Epiduralraumes relativ zum Rückenmarknervengewebe, das stimuliert werden soll, positioniert ist, sodass der Außenmantel einen Gang zum Einführen der elektrischen Stimulationsleitung bildet, wenn der innere Eindringkörper sich entlang dem Führungsdraht nach vorne bewegt, und wobei der innere Eindringkörper so beschaffen ist, dass er vom Außenmantel entfernt werden kann, wodurch der Außenmantel im Wesentlichen in der Stellung im Epiduralraum belassen wird, um die elektrische Stimulationsleitung durch den Außenmantel in eine Position in der Nähe des Rückenmarknervengewebes, das stimuliert werden soll, einzuführen;
und einem implantierbaren Generator, um die elektrische Stimulationsleitung mit Energie zu versorgen;
**dadurch gekennzeichnet, dass** die Spitze des inneren Eindringkörpers und die Spitze des Außenmantels einen gekrümmten Teil beinhalten, um den Durchgang des inneren Eindringkörpers und des Außenmantels um ein anatomisches Hindernis herum zu ermöglichen.

## Revendications

1. Introducteur destiné à l'implantation d'une dérivation de stimulation électrique pour permettre la stimulation électrique d'un tissu nerveux, comprenant :
une gaine externe conformée pour accepter l'insertion de la dérivation de stimulation électrique à travers la gaine externe, cette dernière pouvant être insérée dans un corps humain à proximité du tissu nerveux ; et
un pénétrateur interne placé de manière amovible à l'intérieur de la gaine externe et comprenant un canal interne conformé pour accepter un fil guide, le pénétrateur interne étant conformé pour être avancé le long du fil guide jusqu'à un emplacement souhaité relativement au tissu nerveux et retiré de la gaine externe en laissant la gaine externe sensiblement en position pour l'insertion de la dérivation de stimulation électrique à travers la gaine externe à une position à proximité du tissu nerveux, **caractérisé en ce que** la pointe du pénétrateur interne et la pointe de la gaine externe possèdent une partie incurvée pour permettre le passage du pénétrateur interne et de la gaine externe autour d'une obstruction anatomique.

2. Introducteur de la Revendication 1, dans lequel la dérivation a une largeur supérieure à 1,5 millimètres.

3. Introducteur de la Revendication 1, dans lequel la dérivation de stimulation électrique a une largeur d'environ au moins deux fois sa hauteur.

4. Introducteur de la Revendication 1, dans lequel la dérivation de stimulation électrique est une dérivation de style palette.

5. Introducteur de la Revendication 1, dans lequel le tissu nerveux comprend du tissu nerveux rachidien et l'emplacement souhaité comprend un espace épidural de l'être humain.

6. Introducteur de la Revendication 1, dans lequel le tissu nerveux comprend un nerf périphérique.

7. Introducteur de la Revendication 1, dans lequel le pénétrateur interne comprend une pointe creuse conformée pour s'étendre au-delà de la gaine externe.

8. Introducteur de la Revendication 1, dans lequel la gaine externe comprend une arête saillante circonférentielle conformée pour créer une résistance lorsque l'arête circonférentielle entre en contact avec le ligament jaune de l'être humain.

9. Introducteur de la Revendication 1, dans lequel le pénétrateur interne et la gaine externe comprennent un marqueur radio-opaque ou davantage pour une visualisation à la radioscopie.

10. Introducteur de la Revendication 1, dans lequel la gaine externe possède une section croisée sensiblement ovale.

11. Introducteur de la Revendication 1, dans lequel la gaine extérieure possède une section croisée sensiblement oblongue.

12. Introducteur de la Revendication 1, dans lequel la gaine externe est formée d'un métal renfermant du titane ou de l'acier inoxydable.

13. Introducteur de la Revendication 1, dans lequel le pénétrateur interne est formé d'un ou de plusieurs matière(s) plastique(s), silastique(s) ou polymérique(s).

14. Introducteur de la Revendication 1, dans lequel la pointe du pénétrateur externe est conique.

15. Introducteur de la Revendication 1, dans lequel le pénétrateur interne est conformé pour être avancé jusqu'à ce qu'une extrémité du pénétrateur interne soit positionnée dans l'espace épidural de l'être humain à un emplacement souhaité relativement au tissu nerveux de telle sorte que la gaine externe forme un passage d'insertion pour la dérivation de stimulation électrique à mesure que le pénétrateur interne avance le long du fil guide.

16. Introducteur de la Revendication 1, dans lequel la pointe de l'introducteur est conique et creuse.

17. Introducteur de la Revendication 1, dans lequel la gaine externe comprend une arête circonférentielle conformée pour créer une résistance lorsque l'arête circonférentielle entre en contact avec un ligament jaune d'être humain.

18. Introducteur de la Revendication 1, dans lequel l'aiguille comprend un stylet amovible conformé pour être retiré avant l'insertion du fil guide.

19. Système destiné à l'implantation d'une dérivation de stimulation électrique pour permettre la stimulation électrique d'un tissu nerveux rachidien d'être humain, comprenant :
une aiguille ;
un fil guide ;
un introducteur comportant une gaine externe et un pénétrateur interne ;
la gaine externe étant conformée pour accepter l'insertion de la dérivation de stimulation électrique à travers la gaine externe, cette dernière étant exploitable pour une insertion à travers la peau humaine et dans l'espace épidural humain ;
un pénétrateur interne placé de façon amovible à l'intérieur de la gaine externe et comprenant un canal interne conformé pour accepter un fil guide ;
le pénétrateur interne conformé pour être avancé le long du fil guide jusqu'à une extrémité du pénétrateur interne est positionné dans l'espace épidural à un emplacement souhaité relativement au tissu du nerf rachidien à stimuler de manière à ce que la gaine externe forme un passage d'insertion pour la dérivation de stimulation électrique à mesure que le pénétrateur interne avance le long du fil guide, le pénétrateur interne étant conformé pour être retiré de la gaine extérieure en laissant la gaine extérieure sensiblement en position dans l'espace épidural pour l'insertion de la dérivation de stimulation électrique à travers la gaine externe en position à proximité du tissu nerveux rachidien à stimuler ; et un générateur implantable pour alimenter la dérivation de stimulation électrique, **caractérisé en ce que** la pointe du pénétrateur interne et la pointe de la gaine externe possèdent une partie incurvée pour permettre le passage du pénétrateur interne et de la gaine externe autour d'une obstruction anatomique.
